(19) Europäisches Patentamt

European Patent Office

Office européen des brevets

(11) Veröffentlichungsnummer: **0 647 708 A2**

## (12) EUROPÄISCHE PATENTANMELDUNG

(21) Anmeldenummer: **94114133.5**

(51) Int. Cl.⁶: **C12M 1/26**, C12C 11/02

(22) Anmeldetag: **08.09.94**

(30) Priorität: **15.09.93 DE 4331409**

(43) Veröffentlichungstag der Anmeldung:
**12.04.95 Patentblatt 95/15**

(84) Benannte Vertragsstaaten:
**AT BE CH DE DK FR GB IT LI NL**

(71) Anmelder: **BAM Apparate-und Maschinenbau GmbH**
**Industriestrasse 2**
**D-85405 Nandlestadt (DE)**

(72) Erfinder: **Back, Werner, Prof. Dr.**
**An der Mühle 9a**
**D-85354 Freising (DE)**
Erfinder: **Breuer, Dieter**
**Ferd.-Zwack-Strasse 3**
**D-85354 Freising (DE)**

(74) Vertreter: **Riederer Freiherr von Paar zu Schönau, Anton**
**Lederer, Keller & Riederer,**
**Postfach 26 64**
**D-84010 Landshut (DE)**

(54) **Verfahren und Anlage zur Vermehrung von Hefe.**

(57) Bei der Vermehrung von Hefe, insbesondere Bierhefe, wird in einem tankartigen Assimilator (1, 1, 1'') eine Grundmenge Hefe mit als Nahrung der Hefe dienender Nährlösung, insbesondere Würzelösung, zu einer Suspension gemischt, die im Bereich der Rohrleitung mit gelöstem Sauerstoff angereichert wird (in 11) und der schubweise neue Nährlösung zugeführt wird, und vermehrt sich in diesem Milieu bei geregelter Temperatur bis zu einer vorgegebenen Populationsdichte, woraufhin die Suspension teilweise oder ganz aus dem Assimilator abgezogen und einem Gärtank zugeführt wird; hierbei mißt man eine vom Extraktgehalt der Suspension abhängige Meßgröße (in 43, 45) und den Sauerstoffgehalt (in 41) der Suspension die Rate der $CO_2$-Entstehung (in 39, 40) und hält die Gehalte in für das jeweilige Vermehrungsprogramm festgelegten Grenzen, so daß die Hefevermehrung gezielt in der logarithmischen Phase gehalten wird. Eine hierzu verwendbare Anlage umfaßt wenigstens einem tankartigen Assimilator (1, 1', 1''), der über eine Heiz/Kühl-Einrichtung (23, 25) und eine Inhaltsmengen-Meßvorrichtung (37) verfügt und einen Einlaß- und einen Auslaßanschluß (3, 3''; 5, 5'') mit jeweiligem Anschlußrohr (7, 7', 7'') aufweist, von denen das Einlaß-Anschlußrohr (über 15) Verbindung zu einer Nährlösung-Zudosiervorrichtung hat und eines der Rohre in seinem Verlauf eine Sauerstoff-Zudosiervorrichtung (11) durchläuft, und ist dadurch gekennzeichnet, daß sich im Assimilator (1, 1', 1'') und/oder einem hiermit verbundenen Rohr Meßsonden (39, 40, 41, 43, 45) für eine vom Extraktgehalt der Suspension abhängige Meßgröße und für den Gehalt an gelöstem Sauerstoff oder für die Zunahmerate des $CO_2$ befinden.

Fig. 1

EP 0 647 708 A2

Die Erfindung bezieht sich auf ein Verfahren nach dem Oberbegriff des Anspruchs 1 und eine Anlage nach dem Oberbegriff des Anspruchs 7 zur Vermehrung von Hefe, insbesondere Bierhefe. Die Grundsätze des Verfahrens und der Grundaufbau der Anlage sind jedoch auch für die Vermehrung von Wein- und Backhefe anwendbar.

Wird in Brauereien für den Gärprozeß im Gärbehälter jeweils Hefe verwendet, die aus einem oder mehreren vorhergehenden Gärprozessen stammt, so nimmt die Gäraktivität der Hefe aus verschiedenen Gründen allmählich ab, beispielsweise auf Grund einer Anreicherung mit geschwächten oder abgestorbenen Hefezellen oder durch Überalterung und mangelndem Stoffwechselaustausch, durch Degenerationserscheinungen oder auch durch Überlagerung mit schädlichen Fremdorganismen - Kontamination.

Es ist bekannt (DE-PS 4137537; 19. Brautechnische Arbeitstagung v. 22.-24.10.92 in Alpirsbach, Prof. Dr. W. Back "Hefeführung, Hefepflege und Einfluß auf die Bierqualität", sowie Vortrag am 13.11.92, anläßlich der Brau'92 in Nürnberg zu "Verbesserung der Gärkraft und Widerstandsfähigkeit von Brauerei-Kulturhefen"; BRADWELT 16/1993, S. 646-649), daß es für den Gärprozeß förderlich ist, wenn die Anstellhefe neu hergeführt oder aktiviert wird. Die für die Hefeerzeugung verwendete Anlage umfaßt einen Vermehrungstank, dem ursprünglich eine gewisse Menge Reinzuchthefe eingegeben wurde und dem außerdem Sterilwürze eingespeist wird. Der Vermehrungstank wird auf Parameter eingestellt, die von den optimalen Gärungsparametern abweichen, jedoch für die Vermehrung der Hefezellen geeignet sind. Zur Einstellung der Parameter ist beim Stand der Technik außerhalb des Vermehrungstanks ein Kreislauf vorgesehen, durch den die Würze-Hefe-Suspension umgewälzt wird, wobei in diesem Kreislauf der Suspension Sterilluft und weitere Sterilwürze unter Einhaltung eines gegebenen Mischungsverhältnisses zugeführt werden.

Durch die Erfindung soll die Hefevermehrung in der vom Gärtank getrennten Anlage zur Vermehrung der Hefe optimiert werden. Dies wird durch das im Anspruch 1 gekennzeichnete erfindungsgemäße Verfahren erreicht, das vorzugsweise mit der im Anspruch 7 gekennzeichneten erfindungsgemäßen Anlage durchgeführt wird. Demnach werden der Extraktgehalt und der Sauerstoffgehalt der Suspension bzw. die $CO_2$-Bildung laufend oder zeitlich verteilt überwacht und entsprechend dem Meßergebnis Sauerstoff schneller oder langsamer nachgespeist und hierdurch der Sauerstoffgehalt in festgelegten Grenzen gehalten, die allerdings je nach Biertyp, Hefe und Bedürfnissen der Brauerei unterschiedlich vorgegeben sein können. Als "Extrakt" wird hierbei in Übereinstimmung mit der Fachsprache die Konzentration verschiedener gelöster Substanzen, insbesondere der von der Hefe verwertbare Zucker in der Würze bezeichnet. Der Gehalt an solchen Zuckern kann direkt durch eine entsprechende Extraktmessung oder indirekt durch Ermittlung des pH-Verlaufes festgestellt werden. Der Extraktgehalt in Verbindung mit dem Füllungsgrad des Assimilators, der Sauerstoffgehalt und die Temperatur werden vorzugsweise gemeinsam geregelt, mit dem Ziel, eine ständige logarithmische Vermehrung der Hefe zu gewährleisten.

Die "logarithmische Phase" stellt die eigentliche Vermehrungsphase bei der Hefekultivierung dar. Man unterscheidet nämlich zwischen einer sog. stationären Phase, einer Anlauf- = Lag-Phase, einer logarithmischen Wachstumsphase und der Absterbephase. Bei der logarithmischen Phase findet eine exponentielle Vermehrung statt, die in kurzer Zeit zu einem massiven Zellzuwachs führt. Der Fachausdruck "logarithmisch" veranschaulicht, daß die Zahl der lebenden Zellen je Zellteilungsgeneration verdoppelt wird.

Insbesondere bei obergäriger Hefe kann die Aktivität so hoch sein und damit der Gehalt an gebundenem Sauerstoff so schnell absinken, daß die Messung des verbliebenen Restbestandes seine Messung schwierig wird. Anstelle dieses Meßwerts kann auch die Entstehung des bei der Gärung bekanntlich erzeugten Kohlendioxidgases als Regelungskriterium herangezogen werden, am einfachsten durch direkte $CO_2$-Gas-Messung in der Suspension oder durch eingeschobene Intervalle, in denen man nach hermetischem Abschluß des Assimilators in diesem den Druckanstieg mißt und nach den daraus gewonnenen Meßwerten die Sauerstoffzugabe steuert.

Die Zellvermehrung in der logarithmischen Phase kann bei niedrigen Temperaturen auch längere Zeit in Anspruch nehmen. Zellen aus der logarithmischen Phase - sowohl bei niedrigen wie bei höheren Wachstumstemperaturen - haben in mehrerlei Hinsicht vorteilhafte Auswirkungen: Die Hefe hat eine derartige Aktivität, daß für die Hauptgärung eine wesentlich niedrigere Menge Anstellhefe genügt. Außerdem kann zur Beschickung des Vermehrungstanks, der in der vorliegenden Ausführung als "Assimilator" zu bezeichnen ist, gewöhnliche Betriebswürze verwendet werden. Die logarithmische Hefe ist derartig aktiv und widerstandsfähig und übt einen so starken Selektionsdruck aus, daß Fremdkeime durch die eigene starke Vermehrung verdrängt werden. Somit ist eine Verwendung von speziell sterilisierter Würze nicht notwendig. Der Vorteil, daß nur eine verminderte Hefemenge erforderlich ist, um die vollständige Gärung durchzuführen, hat auch den nützlichen Effekt, daß insgesamt weniger Althefe entsorgt werden muß. Erntehefe wurde u.a. früher vielfach als Viehfutter verwendet, wird aber in jüngerer Zeit für diesen Zweck zunehmend nur mehr beschränkt angenommen. Die Brauereien stehen je nach Standort auf Sicht gesehen vor Entsorgungsproblemen, die durch die Erfindung gemildert werden können. Die bislang üblichen, großen Mengen Anstellhefen wurden deshalb benötigt, weil ein Teil der Hefezellen in abgeschwächtem oder abgestorbenem Zustand vorlag. Letztere haben auch häufig einen unreinen Geschmack infolge Autolyseerscheinungen und Gärungsnebenprodukten nach sich gezogen und außerdem durch verstärkte Abgabe von Fettsäuren die Schaumhaltigkeit des Bieres beeinträchtigt.

Die richtige Sauerstoffversorgung ist eine Voraussetzung für die logarithmische Vermehrung. Bei zu wenig Sauerstoff in der Suspension beginnt die Hefe zu gären. Eine zu große Menge an Sauerstoff ist hingegen schädlich sowohl hinsichtlich der Tendenz einer Oxidation bestimmter Würzeinhaltsstoffe, als auch wegen der Gefahr, daß die Hefezellen durch Luftblasen vom Nährboden - der Würze - verdrängt werden und so die Nahrungsaufnahme aus der Würze beeinträchtigt wird.

Insbesondere ist das erfindungsgemäße Verfahren nach Anspruch 6 so durchführbar, daß man über einen längeren Zeitraum in regelmäßigen Abständen, die von der Sudfrequenz der Brauerei für den treffenden Biertyp abhängt, einen gegebenen Prozentsatz der Suspension aus einem Assimilator zum Gärtank abzieht und den Assimilator in einer oder in mehreren aufeinanderfolgenden Stufen so, daß die Extraktkonzentration im optimalen Bereich bleibt, unter Aufrechterhaltung der optimalen Sauerstoffkonzentration wieder auffüllt, woraufhin sich die im Assimilator zurückgebliebenen Hefezellen nach wie vor in logarithmischer Phase weitervermehren, bis zum nächsten Mal der gegebene Prozentsatz abgezogen wird. Dieser Prozentsatz kann beispielsweise bei untergäriger Hefe 10% und bei obergäriger Hefe 50% des Arbeitsvolumens, also des Assimilatorvolumens betragen. Für das Nachlegen von Extrakt ist dabei auch die im Assimilator schon vorhandene Suspensionsmenge bedeutsam, die die Hefezellendichte in der Suspension bestimmt. Bei zu niedriger Dichte ist der Selektionsdruck der Hefe nicht mehr optimal.

In größeren Zeitabständen kann die Vermehrung mit einem neuen Stamm von Reinzuchthefe stets neu gestartet werden, wobei die Zudosierung der Würze anfangs in kleineren Stufen erfolgt, um keine zu starke Verdünnung der Hefezellen zu bewirken.

Einzelne längere Perioden, beispielsweise die Sudpause über das Wochenende, können durch Einstellen der Parameter für eine langsamere, aber gleichwohl logarithmische Vermehrung überbrückt werden, so daß die für den nächsten Gärprozeß erforderliche Hefemenge erst zur gegebenen Zeit verfügbar ist und dann nach wie vor die frische ungebrochene Aktivität aufweist. Die für eine solche Verzögerung am einfachsten gezielt zu verändernden Parameter sind Temperatur und Mischungsverhältnis.

Das erfindungsgemäße Verfahren ist durchführbar mit einer Anlage nach Anspruch 7, die vorzugsweise eine Regelungsautomatik enthält. Eine $CO_2$-Meßsende in der Suspension oder ein Druckmeßgerät insbesondere in der oben im Assimilator befindlichen Gasblase können laufend und um die Zeit des Abschlusses der Vermehrung die $CO_2$-Zunahme oder den Gasdruckanstieg messen, der von der Erzeugung von $CO_2$ auf Grund der Hefeaktivität herrührt und eine Anzeige für die Hefeaktivität und somit den Sauerstoffbedarf ergibt. Aus dem $CO_2$-Gasanstieg oder dem $CO_2$-Druckanstieg kann außerdem beispielsweise erkannt werden, ob die Gärung im Gärtank, der mit der Hefe beschickt wird, eine etwas kürzere oder etwas längere Zeit in Anspruch nehmen wird. Die in Anspruch 7 genannte Sauerstoff-Zudosiervorrichtung, die Sterilluft einbringt, kann dadurch ergänzt sein, daß einem speziellen Belüftungsrührwerk im Assimilator eine Luft-Einblasdüse zugeordnet ist. Mit dieser Düse kann beispielsweise das Sauerstoffniveau im erforderlichen Bereich gehalten werden, während eine im Rohrverlauf befindliche, z.B. Venturidüse, die eigentliche Belüftungseinrichtung darstellt. Diese und die Tankinnenbelüftung in Verbindung mit dem Belüftungsrührwerk mit optimalem Luft-Verwirbelungseffekt sind durch regelbare Intervallsteuerung harmonisch aufeinander abgestimmt und CIP-integriert.

In der an sich bekannten Weise kann der einzelne Assimilator für sich allein arbeiten und mit einer externen Verrohrung ausgestattet sein, in dem der erforderliche Sauerstoff in Form von Sterilluft - gegebenenfalls neben dem im Assimilator eingegebenen Sauerstoff - zudosiert wird.

Die bevorzugte Anlage nach Anspruch 10 verwendet hingegen zwei Assimilatoren, in Erweiterung der Anlage läßt sich diese Zahl noch vergrößern. Die Anlage kann so betrieben werden, daß durch Steril-Druckluft, die in einen der Assimilatoren eingebracht wird, die Suspension in den anderen Assimilator umgedrückt wird und auf diesem Weg die Sauerstoff-Dosage erfolgt. Diese Ausführung hat den Vorteil, daß Pumpen vermieden werden, die schnell zu einer Schädigung der Hefezellen führen können. Üblicherweise werden Kreiselpumpen verwendet, die sowohl durch Kavitation als auch durch Scherung eine Gefährdung für die Hefezellen bringen können. Die Konstruktion nach Anspruch 10 vermeidet durch einen pumpenfreien Betrieb im Umdrückverfahren diese Gefährdung. Die ergänzende Maßnahme, daß die Assimilation jeweils in ihrem oberen Bereich mit einer steuerbaren Druckluftquelle in Verbindung stehen, dient dazu, das Umdrückverfahren auch dann durchführen zu können, wenn kurz nach der ersten Beschickung mit Reinzuchthefe erst ein sehr niedriger Suspensionspegel erreicht ist.

Die Anlage nach Anspruch 12 bedient sich zwar wieder einer Pumpe, arbeit aber insgesamt nach dem Prinzip kommunizierender Röhren ebenfalls mit zwei Assimilatoren, die dann zum Zweck des Anzapfens für das Anstellen und zum Zweck des Nachfüllens mit Würze getrennt werden können, so daß diese Vorgänge bei beiden Assimilatoren gleichzeitig vorgenommen werden können. Man kann also gleichzeitig nachladen und abziehen, woraufhin man über die die Ausgangsanschlüsse verbindende Leitung wieder ausgleicht.

Die Maßnahme nach Anspruch 13 betrifft das vollständige Inlösunggehen des eingebrachten Sauerstoffs, bevor die Suspension in den Assimilator zurückfließt. Hierdurch werden ein Perlen der Luft und eine unerwünschte Schaumbildung vermieden.

Weitere Einzelheiten, Vorteile und Weiterbildungen der Erfindung ergeben sich aus der folgenden Beschreibung bevorzugter Ausführungs- und Durchführungsbeispiele unter Bezugnahme auf die Zeichnung. Es zeigen:

Fig. 1    ein Schema eines Einzel-Assimilators zur Durchführung des erfindungsgemäßen Verfahrens;

Fig. 2    ein Schema von zwei zusammengeschalteten Assimilatoren zur Durchführung eines Umpumpens der Suspension;

Fig. 3    ein Schema von zwei zusammengeschalteten Assimilatoren zur Durchführung des Umdrückens der Suspension;

Fig. 4    ein Schema zur Erläuterung des Zusammenschaltens einer größeren Zahl von Assimilatoren;

Fig. 5    ein Schema entsprechend Fig. 4 mit einer abgewandelten Installation;

Fig. 6    ein Schema mit zwei zusammengeschaltenen Assimilatoren in im Vergleich zu Fig. 2 und Fig. 3 abgewandelter Ausführung.

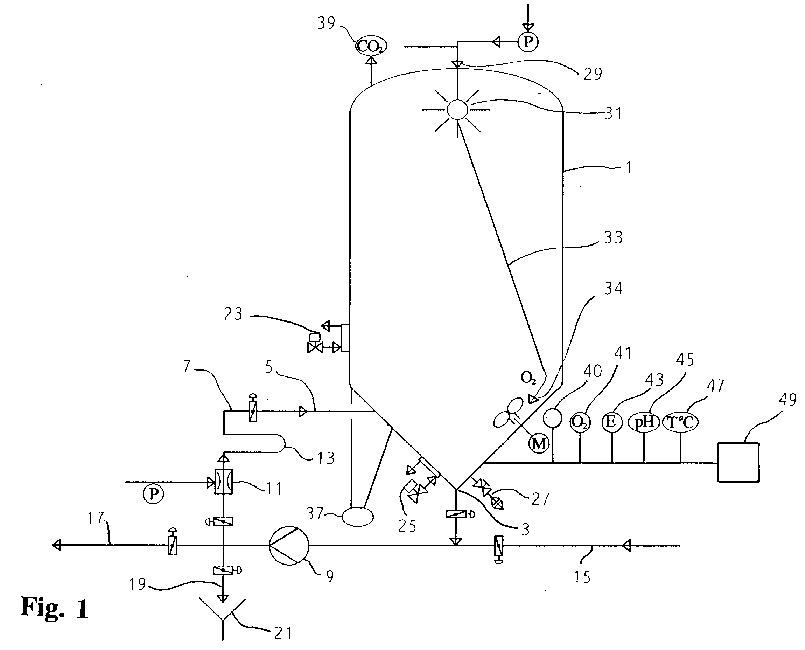

Fig. 1 zeigt einen einzelnen als Assimilator 1 bezeichneten Hefevermehrungstank in Form von eines im wesentlichen geschlossenen Behälters eines Volumens von beispielsweise 30 bis 100 hl für obergärige Hefe oder z.B. 500 hl für untergärige Hefe. Die Behälterform des Assimilators läuft unten konisch auf einen Auslauf zusammen, an dem ein erster Anschluß 3 gebildet ist. Etwas höher im konischen Bereich mündet in den Assimilator ein zweiter Anschluß 5. Zwischen den Anschlüssen 3 und 5 liegt eine Rohr-Ringleitung 7, in deren Verlauf eine Pumpe 9, eine Luft-Zudosiervorrichtung 11, z. B. in Form eines Venturirohres, das Sterilluft P ansaugt, ein dieser Zudosiervorrichtungen nachgeschaltetes mindestens 4 Meter langes Rohrstück, das als $O_2$-Lösungsstrecke 13 dient, und eine Anzahl von Absperrventilen eingeschaltet sind. In diesem durch die Ringleitung 7 gebildeten externen Kreislauf dient der erste Anschluß als Auslaß aus dem Assimilator und der zweite Anschluß als Einlaß in den Assimilator.

In die Ringleitung 7 mündet nach einem dem ersten Anschluß 3 folgenden Absperrventil und vor der Pumpe 9 wiederum über ein Absperrventil eine Würzeleitung 15, die von einer nicht dargestellten würze-Zudosiervorrichtung oder einer sonstigen Würzequelle kommt. Ein Würze-Steriltank, der die Würze keimfrei zwischenspeichert, ist nicht erforderlich. Am Pumpenausgang zweigen von der Ringleitung 7, jeweils über ein Absperrventil, eine Hefe/Würze-Suspensionsleitung 17 und eine Abflußleitung 19, die in einen Gully 21 mündet, ab.

Der Assimilator verfügt über eine Kühlung 23 und, was im Fall der Aufstellung des Assimilators in einem kühlen Gärkeller erforderlich sein kann, über eine Heizung 25. Diese Installationen können auch kombiniert sein. Im Bereich des unteren Endes weist er einen Impfanschluß 27 auf, der zu einem Carlsbergkolben führt, aus dem der Assimilator zum Starten der Vermehrung mit einer gewissen Dosis, beispielsweise 500 bis 1000 g, Reinzuchthefe geimpft werden kann. Der Impfanschluß 27 kann auch für probenentnahmen verwendet werden.

Im oberen Bereich des Assimilators mündet ein gesteuerter Gasdurchlaß 29 mit einer Reinigungsspritzkugel 31, durch die Sterilluft P in den Assimilator durch Druck eingebracht werden kann. Das Eindrücken von Sterilluft über den Gasdurchlaß 29 dient zum Vorspannen des Assimilators zur $O_2$-Blasen-Reduzierung oder zum Umdrücken. Durch den Gasdurchlaß 29 kann andererseits beim Ansteigen des Flüssigkeitspegels im Assimilator Gas nach außen gedrückt werden.

Von der an den Gasdurchlaß 29 anschließenden durch die Spritzkugel 31 geführten Luftleitung 33 wird die Sterilluft im Assimilator nach unten geführt, wo sie über eine Ausblasdüse 34 harmonisierend im Bereich eines speziellen Belüftungsrührwerks 35 mündet, das von einem Motor M mit magnetischer Kraftübertragung durch die Wand hindurch angetrieben wird. Das Rührwerk 35 ist mit einem nicht dargestellten Trockenlaufschutz ausgestattet, der seinen Antrieb verhindert, wenn der Flüssigkeitsspiegel im Assimilator 1 nicht vollständig über dem Rührwerk liegt.

Der Assimilator 1 ist mit folgenden Meßvorrichtungen ausgestattet:
Eine Wägevorrichtung 37 dient primär der Messung des Gewichts des Assimilators, sekundär jedoch der Bestimmung seines Füllungsgrades. Ein Druckmeßgerät 39, nämlich ein elektronisches hochempfindliches Feindruckmeßgerät, mißt den Druck in der über dem Flüssigkeitsspiegel stehenden Gasblase und wird speziell dazu verwendet, in Intervallen, in denen der Gasdurchlaß 29 geschlossen ist, den Druckanstieg über der Zeit zu ermitteln. Insoweit entspricht die Ausstattung noch dem Stand der Technik.

Der Assimilator verfügt weiterhin über eine Meßsonde 40 zur Messung des $CO_2$-Gehalts und eine Meßsonde 41 zur Messung des Sauerstoffgehalts der als Flüssigkeit im Assimilator befindlichen Suspension der Hefe in der Würze, ein Extraktmeßgerät 43, das die Extraktkonzentration in dieser Suspension mißt, einen pH-Wert-Messer 45 und einen Temperaturmesser 47. Das Extraktmeßgerät 43 und der pH-Wert-Messer 45 sind alternative Sensoren. Es ist nur eines der beiden Meßgeräte erforderlich, da der pH-Wert der Suspension sich meist parallel zur Extraktabnahme verhält. Die Verwendung des pH-Wert-Messers ist insofern bevorzugenswert, als derartige handelsübliche Meßgeräte wesentlich kostengünstiger sind als Extraktmeßgeräte. Die Meßgeräte 40, 41, 43, 45 und 47 können Sonden im Inneren des Assimilators 1 und/oder in damit verbundenen Rohren sein.

Mit dieser Anlage kann die Hefevermehrung nach dem folgenden Verfahren durchgeführt werden. Es wird zunächst auf obergärige Hefe Bezug genommen. Zum Start der Anlage wird über den Impfanschluß 27 eine Menge von beispielsweise 500 bis 1000 g Reinzuchthefe eingegeben und wird über die Würzeleitung 15 eine Menge von beispielsweise 15 l Würze eingepumpt, um in möglichst kurzer Zeit die gewünschte Hefedichte zu erreichen. In der zunächst vorhandenen Suspension von also z.B. 1000 g Hefe in 15 l Würze vermehrt sich die Hefe und verbraucht hierbei teilweise den Extrakt der Würze und außerdem Sauerstoff, der in der Würze gelöst ist. Zur Erhaltung des Sauerstoffgehalts wird in an sich bekannter Weise (DE-PS 4137537) von Zeit zur Zeit, beispielsweise im Stundentakt, die Pumpe 9 eingeschaltet und Suspension durch die Ringleitung 7 gepumpt, wo sie in der Luft-Zudosiervorrichtung 11 mit Luft, von der der Sauerstoff ausgenützt wird, angereichert wird. Die Lösungsstrecke 13 dient der vollständigen Auflösung der eingeleiteten Luft, bevor die Suspension in den Assimilator 1 zurückgelangt. Im Verlauf der Vermehrung der Hefe in der Suspension erreicht diese eine schließlich wesentlich höhere Populationsdichte, während der Extrakt der Würze verbraucht wird. Es ist möglich, die Hefezellendichte z.B. durch eine Trübungsmessung zu bestimmen und den Wert in der Regelung mitzuberücksichtigen. Nun wird über die Würzeleitung 15 neue Würze eingeleitet und das Suspensionsvolumen im Assimilator 1 so erhöht, daß die Hefekonzentration noch in einem für die Vermehrung günstigen Wertebereich liegt. Wiederum wird schubweise Sauerstoff nachgeladen, wobei, wenn das Rührwerk 35 einmal vollständig innerhalb der Suspension liegt, der Grund-Sauerstoffbedarf intervallweise getaktet über die Luftleitung 33 mit der Düse 34 und die Verwirbelung durch das Rührwerk 35 gedeckt werden kann. Das Rührwerk 35 soll grundsätzlich mitlaufen während des Heizens, des Kühlens, des Belüftens, der Würzezugabe, der Entnahme und bei der CIP.

Die Sauerstoffaufnahme ist abhängig von der Fließgeschwindigkeit der Würze in der Würzebelüftungseinrichtung. Wird nicht intervallweise, sondern stetig umgepumpt, so kann der Sauerstoffgehalt durch Beeinflussung der Fließgeschwindigkeit geregelt werden.

Das Vorsehen des Auslasses am tiefsten Punkt des Assimilators ermöglicht ein Umpumpen auch dann, wenn kurz nach dem Start der Flüssigkeitsspiegel im Assimilator noch niedrig ist.

Der Assimilator 1 ist schließlich weitgehend mit Suspension gefüllt und diese wird bei Erreichen der maximal gewünschten Hefezellendichte nach entsprechender Einstellung der Absperrventile etwa zur Hälfte über die Pumpe 9 und die Suspensionsleitung 17 zum Anstellen in einen Gärtank für die Hauptgärung abgezogen. Anschließend wird der Assimilator 1 über die Würzeleitung 15 wieder aufgefüllt und die Hefevermehrung setzt sich unverändert fort.

Bei diesem Verfahren wird darauf geachtet, daß die Hefe in der Suspension sich in der Phase der "logarithmischen Vermehrung", also einer ständigen exponentialen Vermehrung, befindet. Der Hefe-Anstellzyklus kann dann, wenn auf maximale Frequenz eingesteuert wird, beispielsweise auf ein Anstellen im Vier-Stundentakt erhöht werden, wobei die Hefe so aktiv ist, daß bei der Hauptgärung im Gärtank eine Hefezellendichte von ca. 20% der herkömmlichen Menge genügt, um die gleiche Hauptgärungszeit zu erreichen, wie sie bisher mit nur durchschnittlich aktiver Hefe möglich war. Das Einhalten der ständigen logarithmischen Vermehrungsphase bringt auch den Vorteil, daß zum Auffüllen der Suspension im Assimilator 1 nicht Sterilwürze, sondern gewöhnliche Ausschlagwürze verwendet werden kann, die im Brauereibetrieb beim Würzekühler abgezweigt wird. Eventuell darin enthaltene Keime werden von der hochaktiven Hefe so verdrängt, daß sie im Assimilator alsbald verschwinden.

Diese logarithmische Vermehrung der Hefe erreicht man nun durch eine Optimierung der Bedingungen im Assimilator 1. Hierzu dient ein automatischer Regler 49, der die Meßwerte der verschiedenen Sensoren 37, 39, 40, 41, 43, 45 und 47 verwertet. Die Meßwerte der Sensoren 39, 40 und 41 werden alternativ verwendet. Bei Schwierigkeiten mit der direkten Messung des Sauerstoffgehalts geht der in Abständen über Intervalle von z.B. 5 Minuten gemessene Druckanstieg im Assimilator, gemessen mit dem Sensor 39, oder der Anstieg des $CO_2$-Gehalts in der Suspension, gemessen mit dem Sensor 40, als Regelgröße in die Regelung ein.

Im hier zunächst besprochenen obergärigen Betrieb wird in einem Temperaturbereich von 12 bis 24°C, insbesonders von 19 bis 22°C, gearbeitet. Der Sauerstoffgehalt soll dann in einem mittleren Bereich von 0,5 bis 2 mg pro Liter und der Extraktbereich in einem Bereich über 7% liegen, wobei dem Extraktgehalt nach oben dadurch Grenzen gesetzt sind, daß der Stammwürzegehalt auf Grund der Biertypen begrenzt ist und meist bei 12, maximal bis 16% liegt. Wird nach dem pH-Wert gegangen, so soll dieser zwischen 4 und 6 liegen. Die Anstell-Hefesuspension wird schließlich mit einer Hefezellenzahl von z.B. 70 ... 180 Millionen Zellen/ml abgezogen.

Bei untergäriger Hefe wird die Regelung im Prinzip nach gleichen Grundsätzen vorgenommen, jedoch liegen die Wertbereiche etwas anders. Die Temperatur liegt bei 6 bis 16°C, insbesonders aber im Bereich von 8 bis 14°C. Auf Grund dieser viel niedrigeren Temperaturen vermehrt sich die Hefezellenzahl entsprechend langsamer, mit der Folge, daß für einen gleichen Zyklus des Abziehens von Anstellhefe nur ein kleinerer Prozentsatz, beispielsweise 10% im Takt von 4 Stunden, entnommen werden kann und für eine gleiche Anstellmenge größere Assimilatoren benötigt werden. Der Sauerstoffgehalt liegt im mittleren Bereich bei z.B. 0,3 bis 1 mg pro Liter und der Extraktgehalt soll wenigstens 6%, bzw. der pH-Wert wie bei der obergärigen Hefe 4 bis 6 betragen. Die Anstell-Hefezellenzahl liegt vorteilhaft in einem Bereich von 30 ... 180 Millionen Zellen/ml.

Das Verfahren ist auch z.B. für Dosierhefe für die Weißbier-Flaschengärung mit Vorteil anwendbar.

Die Vermehrung der Hefe in logarithmischer Phase bedeutet nicht, daß stets auf maximale Vermehrungsgeschwindigkeit eingeregelt wird. Die Vorteile der logarithmischen Phase liegen in der Möglichkeit der schnellen Vermehrung, wenn dies gerade gebraucht wird, aber insbesondere in der weit gesteigerten Aktivität der Hefe. Letztere ergibt sich auch, wenn aus betrieblichen Gründen mit einer langsameren Vermehrung gefahren wird, beispielsweise, wenn der nächste Gärprozeß erst nach dem Wochenende, also in zwei oder drei Tagen, ansteht. Bei zu wenig Sauerstoff in der Suspension beginnt die Hefe zu gären. Längere Vermehrungsperioden werden also nicht durch Luftmangel, sondern hauptsächlich durch Temperaturabsenkung, aber auch durch entsprechende Verdünnung der Hefe mit Würze eingeregelt. Zum Anstellen soll die Temperatur der Suspension aber um nicht mehr als 2°C von der Anstelltemperatur des Gärtanks abweichen.

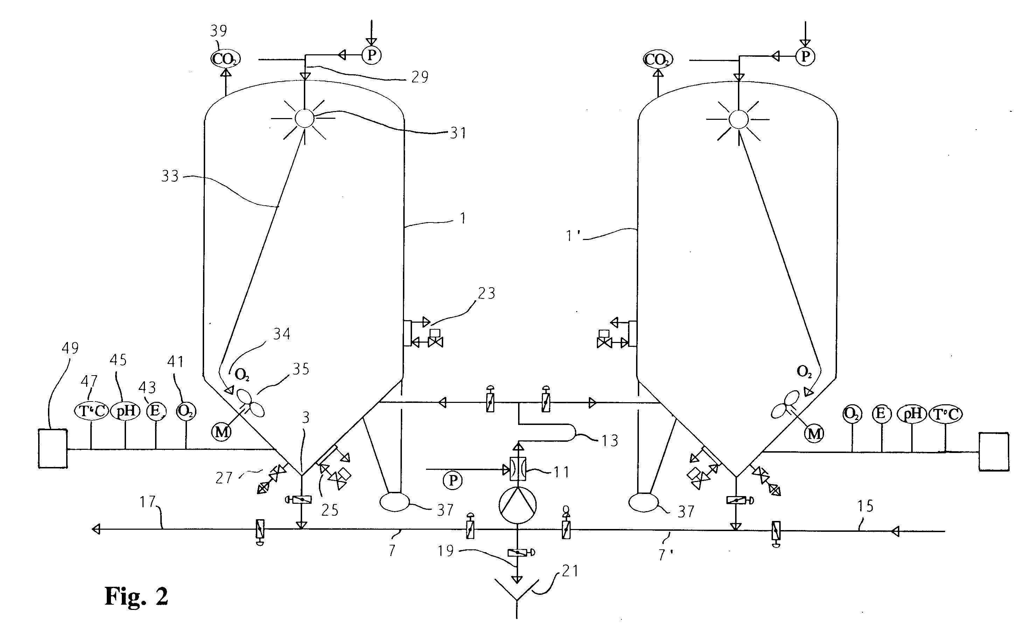

Bei der Darstellung nach den Fig. 2 bis 6 sind die Sensoren 39 und 40 teilweise weggelassen, da sie nicht in jedem Fall benötigt werden.

Fig. 2 zeigt eine Anlage mit zwei Assimilatoren 1 und 1', die dem Assimilator 1 von Fig. 1 gleichen, jedoch in ihrer jeweiligen Ringleitung 7, 7' einen gemeinsamen Zweig mit der Pumpe 9, der Luft-Zudosiervorrichtung 11 und der $O_2$-Lösungsstrecke 13 haben. Eine solche Doppelanordnung eignet sich dann, wenn Anstellhefe in größerer Frequenz benötigt wird und die beiden Assimilatoren 1 und 1' mit Hefe und Ausschlagwürze gleicher Art beschickt werden.

Der Betrieb kann dann in zweierlei Varianten durchgeführt werden, nämlich nach Art von zwei unabhängigen Einzel-Assimilatoren, die jedoch den externen Kreislauf über die Ringleitung 7 bzw. 7' zu verschiedenen Zeiten, insbesondere im Gegentakt, aktivieren. Oder alternativ können die beiden Assimilatoren 1 und 1' auch zusammenwirken, indem das Umpumpen durch die Luft-Zudosiervorrichtung 11 jeweils von einem der Assimilatoren in den anderen erfolgt, also von 1 nach 1' und das nächst Mal von 1' zurück nach 1. Bei einen solchen Betrieb kann, um die Hefezellen zu schonen, diese Pumpe auch ausgeschaltet bleiben und das Durchdrücken durch die Luft-Zudosiervorrichtung 11 und die $O_2$-Lösungsstrecke 13 durch Einpumpen von Sterilluft über die Düse 31 in den zu entleerenden Assimilator, während der jeweils andere Assimilator über den Gasdurchlaß 29 entlüftet wird, erfolgen. Auch das Ableiten der Anstellsuspension wird nicht durch Ingangschalten der Pumpe 9, sondern durch Herausdrücken des gegebenen Prozentsatzes aus dem gefüllten Assimilator bewirkt.

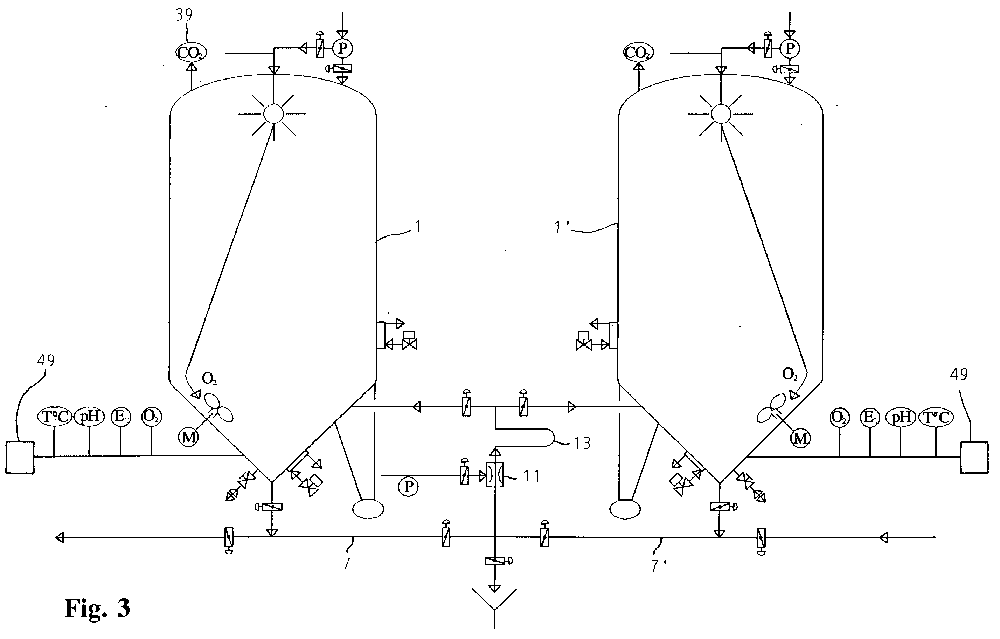

Soll das Umpumpen der Suspension grundsätzlich vermieden werden, so kann die Pumpe 9 auch vollständig weggelassen werden und das intervallweise Durchleiten der Suspension durch die Luft-Zudosiervorrichtung 11 und die $O_2$-Lösungsstrecke 13 grundsätzlich nur durch Umdrücken von einem zum anderen Assimilator erfolgen. Eine entsprechende Anlage ist in Fig. 3 dargestellt.

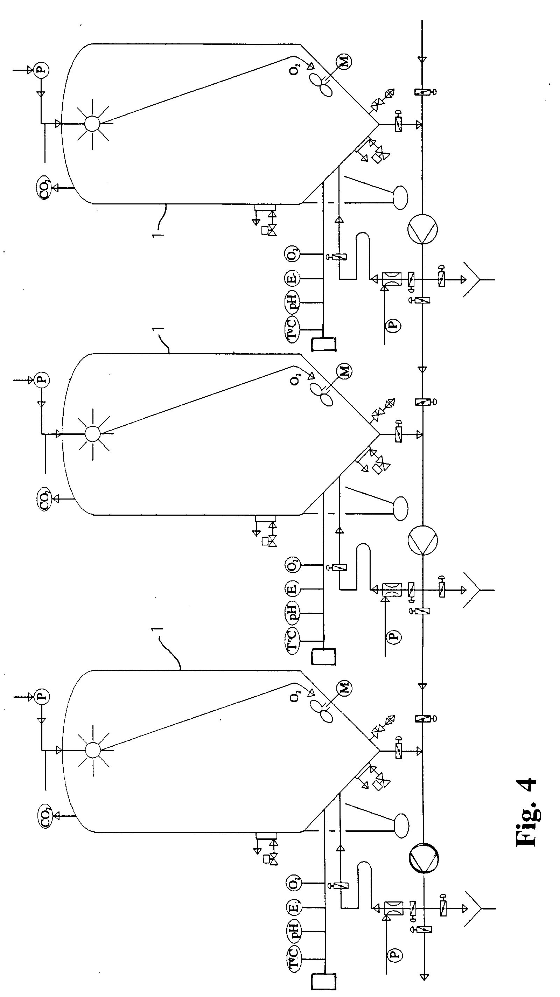

Einfach-Assimilator-Anlagen und Mehrfach-Assimilator-Anlagen lassen sich auch vielfältig kombinieren, wenn es der Brauhefebedarf der Brauerei erfordert. In Fig. 4 ist eine Anlage mit drei einzelnen Assimilatoren 1 dargestellt, die wiederum beispielsweise so variiert werden könnte, daß zwei der Assimilatoren gemäß Fig. 3 geschaltet sind. Mehr-Assimilator-Anlagen sind für größere Brauereien mit schnellerer Sudfolge, aber auch z.B. bei Vermehrung verschiedener Hefestämme geeignet.

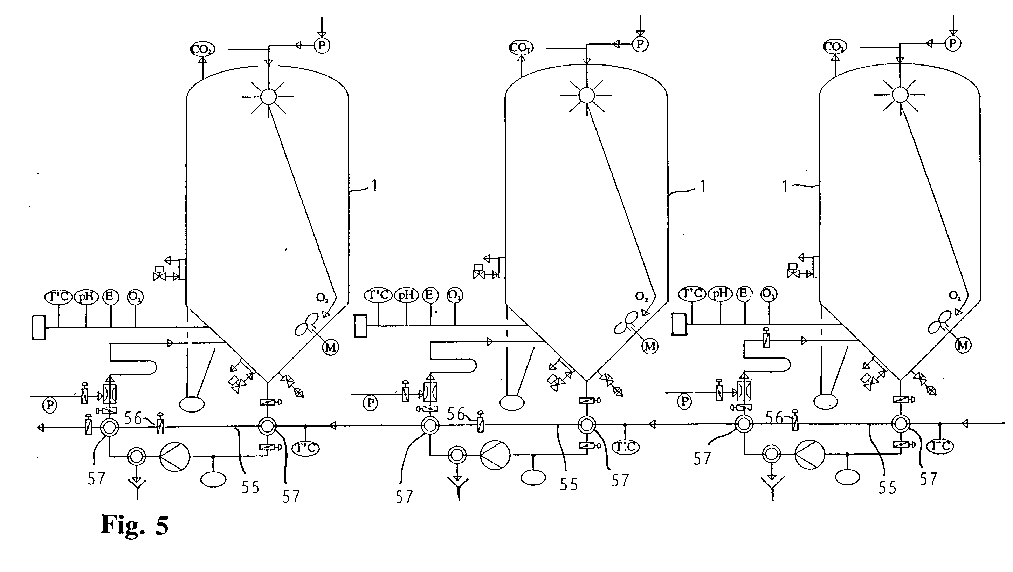

In Fig. 5 ist die Anordnung nach Fig. 4 dahingehend abgewandelt dargestellt, daß dort jeder Assimilator hinsichtlich der Zuführung von Würze und der Ableitung von Suspension zum Gärtank unabhängig von den anderen Assimilatoren ist. Jeder der die Pumpe 9 und die Abflußleitung 19 der einzelnen Assimilatoren enthaltenden Leitungszweige ist durch einen zusätzlichen Leitungszweig 55, der noch ein Absperrventil 56 enthält, überbrückt. An den jeweiligen Abzweigstellen befinden sich Zweiweg-Ventile 57, die in der Darstellung nach Fig. 5 in der einen Ventilstellung in vertikaler Richtung in der anderen Ventilstellung in horizontaler Richtung durchlassen, während sie in der jeweils anderen Richtung sperren.

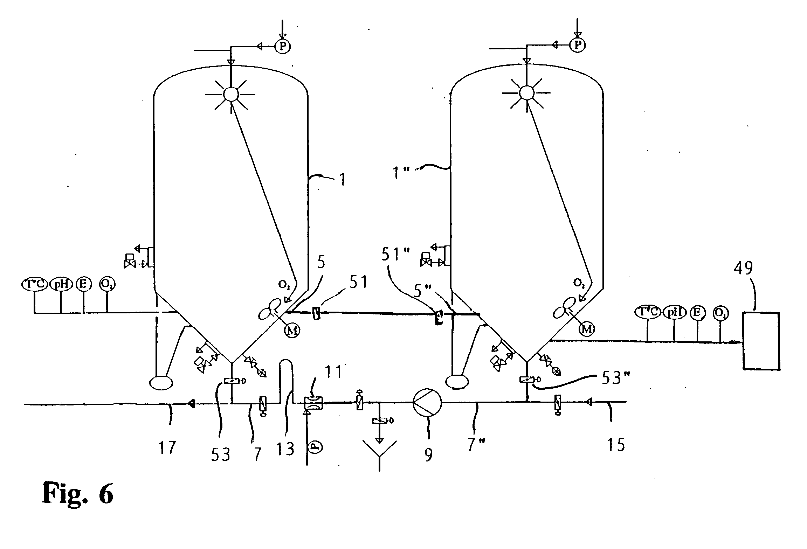

Fig. 6 zeigt eine nochmal abgewandelte Anordnung von zwei Assimilatoren 1 und 1''. Der erste Anschluß 3 bzw. 3'' und der zweite Anschluß 5 bzw. 5'' dienen hier abwechselnd als Einlaßanschluß und Auslaßanschluß. Die zweiten Anschlüsse 5, 5'' sind über Absperrventile 51, 51'' miteinander verbunden, und die ersten Anschlüsse 3, 3'' sind über eine als Ringleitung 7'' bezeichnete Leitung verbunden, die die Pumpe 9, die Luft-Zudosiervorrichtung 11 und die $O_2$-Lösungsstrecke 13 enthält. Absperrventile 53, 53'' trennen die Anschlüsse 3 bzw. 3'' von der Ringleitung 7'', in die vergleichbar der Anordnung nach Fig. 1 die Würzeleitung 15 einmündet bzw. von der die Suspensionsleitung 17 abzweigt, jeweils wiederum unter Zwischenschaltung von Absperrventilen. Über die Verbindung der Anschlüsse 5, 5'' wirken die Assimilatoren 1 und 1'' als kommunizierende Röhren. Der Vorteil der Anlage nach Fig. 5 ist, daß man gleichzeitig nachladen und abziehen kann.

Im einzelnen kann das Verfahren folgendermaßen betrieben werden: Der Sudrhythmus der Brauerei ergibt bestimmte Zeiten für den Bedarf an Anstellsuspension und für den Anfall an Ausschlagwürze. Entsprechend diesem Sudrhythmus wird der Assimilator 1 geladen und auf logarithmische Vermehrung der Hefe eingeregelt. Bis zum nächsten Anfall von Ausschlagwürze bzw. bis zum nächsten Bedarf an Anstellsuspension wird weiter vermehrt, wobei die Suspension durch Umdrücken über die Verbindung der Anschlüsse 5, 5'' in den Assimilator 1'' verbracht wird. Die Vermehrung wird in beiden Assimilatoren auf das Aufrechterhalten der logarithmischen Phase rechnergestützt geregelt. Zur Anreicherung mit Sauerstoff wird zwischen den Assimilatoren über die Ringleitung 7'' umgepumpt und gleichzeitig über die Verbindung der Anschlüsse 5, 5'' ausgeglichen. Schließlich kann nach Schließung der Absperrventile 51, 51'' und bei geöffneten Absperrventilen 53, 53'' gleichzeitig aus dem Assimilator 1 Anstellsuspension abgezogen werden und der Assimilator 1'' mit Würze nachgefüllt werden.

## Patentansprüche

1. Verfahren zur Vermehrung von Hefe, insbesondere Bierhefe, bei dem in einem tankartigen Assimilator (1, 1', 1''), der mit einer Rohrleitung (7, 7', 7'') verbunden ist, eine Grundmenge Hefe mit als Nahrung der Hefe dienender Nährlösung, insbesondere Bierwürze, zu einer Suspension gemischt wird, die im Bereich der Rohrleitung mit gelöstem Sauerstoff angereichert wird (in 11) und der schubweise neue Nährlösung zugeführt wird, und sich in diesem Milieu bei geregelter Temperatur bis zu einer vorgegebenen Populationsdichte vermehrt, woraufhin die Suspension teilweise oder ganz aus dem Assimilator abgezogen und einem Gärtank zugeführt wird, dadurch gekennzeichnet, daß man über die Hefevermehrungszeit eine vom Extraktgehalt der Suspension abhängige Meßgröße (in 43, 45) und den Sauerstoffgehalt (in 41) der Suspension und/oder die Zunahmerate von Kohlendioxid als Gas oder als Druck (in 39) im Assimilator mißt und den Extrakt- und den Sauerstoffgehalt in für das jeweilige Vermehrungsprogramm festgelegten Grenzen hält.

2. Verfahren nach Anspruch 1, dadurch gekennzeichnet, daß man den Gehalt an Nährlösung durch eine Regelung (in 49) mit Rückmeldung des Meßwerts der vom Extraktgehalt der Suspension abhängigen Meßgröße (von 43, 45) automatisch laufend optimiert.

3. Verfahren nach Anspruch 1 oder 2, dadurch gekennzeichnet, daß man auch den Sauerstoffgehalt (von 41) der Suspension und/oder die Zunahmerate des Kohlendioxids automatisch geregelt (in 49) in einem optimierten Bereich hält.

4. Verfahren nach Anspruch 2 oder 3, dadurch gekennzeichnet, daß man die Hefevermehrungsparameter als optimierten Zustand auf eine logarithmische Vermehrung der Hefe, nämlich auf den Zustand der Verdoppelung der Zahl lebender Hefezellen je Milliliter in aufeinanderfolgenden vorgegebenen Zeitspannen einstellt.

5. Verfahren nach einem der Ansprüche 1 bis 4, dadurch gekennzeichnet, daß man als Meßgröße für den Extraktgehalt den pH-Wert der Suspension (in 45) mißt.

**6.** Verfahren nach einem der Ansprüche 1 bis 5, dadurch gekennzeichnet, daß man periodisch im Abstand einiger Stunden einen gegebenen Prozentsatz der Suspension zum Zweck des Anstellens zu einem Gärtank abzieht und anschließend im Assimilator (1, 1', 1'') den Pegel der Suspension durch die geregelte Zugabe von Nährlösung stufenweise wieder erhöht, und daß man zur Verlängerung einzelner Perioden das Verhältnis Nährlösung/Hefe über den optimalen Wert erhöht und/oder die Temperatur unter den optimalen Wert absenkt.

**7.** Anlage zur Durchführung des Verfahrens nach einem der Ansprüche 1 bis 10, mit wenigstens einem tankartigen Assimilator (1, 1', 1''), der über eine Heiz/Kühl-Einrichtung (23, 25) und eine Inhaltsmengen-Meßvorrichtung (37) verfügt und einen Einlaß- und einen Auslaßanschluß (3, 3''; 5, 5'') mit jeweiligem Anschlußrohr (7, 7', 7'') aufweist, von denen das Einlaß-Anschlußrohr (über 15) Verbindung zu einer Nährlösung-Zudosiervorrichtung hat und eines der Rohre in seinem Verlauf eine Sauerstoff-Zudosiervorrichtung (11) durchläuft, dadurch gekennzeichnet, daß sich im Assimilator (1, 1', 1'') und/oder einem hiermit verbundenen Rohr Meßsonden (39, 40, 41, 43, 45 ..) für eine vom Extraktgehalt der Suspension abhängige Meßgröße, und für den Gehalt an gelöstem Sauerstoff und/oder für den Anstieg von Gas oder Druck der Menge von Kohlendioxid befinden.

**8.** Anlage nach Anspruch 7, dadurch gekennzeichnet, daß die Meßsonden (39, 40, 41, 43, 45, 47) des Assimilators (1, 1', 1'') mit einer Regelungsautomatik (49) verbunden sind, die die Heiz/Kühl-Einrichtung (23, 25), die Nährlösung-Zudosiervorrichtung (15) und die Sauerstoff-Zudosiervorrichtung (11) geregelt steuert.

**9.** Anlage nach Anspruch 7 oder 8, dadurch gekennzeichnet, daß sich im Assimilator (1, 1', 1'') ein Belüftungsrührwerk (35) befindet, in dessen Wirkungsbereich eine Luft-Einblasdüse (34) angeordnet ist.

**10.** Anlage nach einem der Ansprüche 7 bis 9, dadurch gekennzeichnet, daß zwei Assimilatoren (1, 1') zusammengeschaltet sind, die jeweils in ihrem oberen Bereich mit einer steuerbaren Druckluftquelle (29, 31) in Verbindung stehen und deren Einlaßanschlüsse (5) über zwei Absperrorgane miteinander verbunden sind und deren Auslaßanschlüsse (3) über zwei Absperrorgane miteinander verbunden sind, und daß die beiden Bereiche zwischen den jeweiligen Absperrorganen durch eine Rohrleitung miteinander verbunden sind, in deren Verlauf die Sauerstoff-Zudosiervorrichtung (11) liegt.

**11.** Anlage nach Anspruch 10, dadurch gekennzeichnet, daß die Assimilatoren (1, 1') am unteren Ende einen sich nach unten zu verjüngenden Querschnitt haben und daß die Auslaßanschlüsse (3) an den untersten Punkten der Assimilatoren und die Eingangsanschlüsse (5) etwas höher liegen.

**12.** Anlage nach einem der Ansprüche 7 bis 9, dadurch gekennzeichnet, daß zwei Assimilatoren (1, 1'') zusammengeschaltet sind, deren Eingangsanschlüsse (3, 3'') über wenigstens ein Absperrorgan (53, 53''), eine Pumpe (9) und die Sauerstoff-Zudosiervorrichtung (11) miteinander verbunden sind und deren Ausgangsanschlüsse (5, 5'') über wenigstens ein Absperrorgan (51, 51'') miteinander verbunden sind, und die jeweils in ihrem oberen Bereich mit einer steuerbaren Druckluftquelle (29, 31) in Verbindung stehen und am unteren Ende einen sich nach unten zu verjüngenden Querschnitt haben, wobei die Einlaßanschlüsse (3, 3'') an den untersten Punkten der Assimilatoren und die Ausgangsanschlüsse (5, 5'') etwas höher liegen.

**13.** Anlage nach einem der Ansprüche 7 bis 12, dadurch gekennzeichnet, daß zwischen der Sauerstoff-Zudosiervorrichtung (11) und jedem der nachfolgenden Anschlüsse (3, 5) eine Rohrlänge (13) von mindestens 4 m liegt.

Fig. 1

EP 0 647 708 A2

Fig. 2

EP 0 647 708 A2

Fig. 3

EP 0 647 708 A2

Fig. 4

EP 0 647 708 A2

Fig. 5

**Fig. 6**

EP 0 647 708 A2